# EUROPEAN PATENT APPLICATION

(11) **EP 2 289 900 A1**
(43) Date of publication of application: **02.03.2011**
(21) Application number: 09168712.9
(22) Date of filing: 26.08.2009
(51) Int. Cl.: C07F 9/38, A61K 31/663, A61P 31/00, A61P 35/00

(54) **Bisphosphonates as inhibitors of acid sphingomyelinase**

(71) Applicant: Humboldt Universität zu Berlin, 10099 Berlin (DE); Rheinisch-Westfälische Technische Hochschule Aachen (RWTH), 52062 Aachen (DE)
(72) Inventor: Arenz, Christoph, 10249 Berlin (DE); Roth, Gundula, 10557 Berlin (DE); Uhlig, Stefan, 52076 Aachen (DE); Drescher, Daniela, 12437 Berlin (DE)
(74) Representative: Gulde Hengelhaupt Ziebig & Schneider

(57) **Abstract**

The present invention refers to bisphosphonate and phosphonate/phosphate compounds of Formulae I and II and its use as inhibitors of aSMase enzyme activity.

## Description

The acid sphingomyelinase (aSMase) is a soluble lysosomal sphingolipid hydrolase, which constitutively degrades sphingomyelin from internalized membrane fragments (T. Kolter, K. Sandhoff, Angew. Chem. 1999, 111, 1632; Angew Chem Int Ed 1999, 38, 1532). Upon stimulation, a portion of this enzyme can be found at the outer side of the plasma membrane (S. Marathe, S. L. Schissel, M. J. Yellin, N. Beatini, R. Mintzer, K. J. Williams, I. Tabas, J. Biol. Chem. 1998, 273, 4081). This membrane-associated enzyme shows biochemical activity in serum and urine. Its activity is elevated in several diseases. The secretory form of aSMase is believed to play an important role in signal transduction, since it alters the composition of the plasma membrane within putative sphingolipid- and cholesterol-rich membrane micro-domains. These so-called 'lipid rafts' have been suggested to act as 'signalling platforms' (K. Simons, E. Ikonen, Nature 1997, 387, 569) and there is significant evidence, that the cleavage of sphingomyelin to ceramide is able to dramatically alter the biophysical properties of the putative rafts (Megha, E. London, J Biol Chem 2004, 279, 9997).

The aSMase is emerging as an important drug target in a variety of diseases. Amongst others, it has been shown that inhibition of aSMase prevents bacterial infections in a rat model of cystic fibrosis and formation of acute lung injury (ALI) elicited by endotoxin, acid instillation or platelet-activating factor (PAF). Moreover, the aSMase is essential for infection of non-phagocytotic cells with *Neisseria gonorrhoea* and formation of pulmonary emphysema. Pharmacological or genetic inhibition of aSMase prevents apoptosis and degeneration of liver cells in a mouse model for Wilson's disease. In addition, there are several reports that aSMase significantly contributes to the formation of atherosclerotic plugs.

However, this promising progress in aSMase-research, based on sophisticated animal models and cultured patient's cells, is thwarted by the lack of potent and selective inhibitors of this enzyme. Phosphatidylinositol-3,5-bisphosphate (Ptdlns3,5P₂), the most potent inhibitor (M. Kolzer, C. Arenz, K. Ferlinz, N. Werth, H. Schulze, R. Klingenstein, K. Sandhoff, Biol Chem 2003, 384, 1293.), is not suited for cell culture studies or straight forward in vivo application, because of its 5-fold negative charge and its two long fatty acid chains causing it to stack in cellular membranes. Last but not least, this inhibitor is labile towards phospholipases A₁, A₂, C and D and phosphoinositide phosphatases.

It is an object of the present invention to provide novel potent inhibitors of acid sphingomyelinase.

It has surprisingly been found that compounds of Formula I and compounds of Formula II are efficient inhibitors of aSMase enzyme activity.

Compounds of Formula I comprise geminal bisphosphonates and mixed phosphonate/phosphate compounds described by: 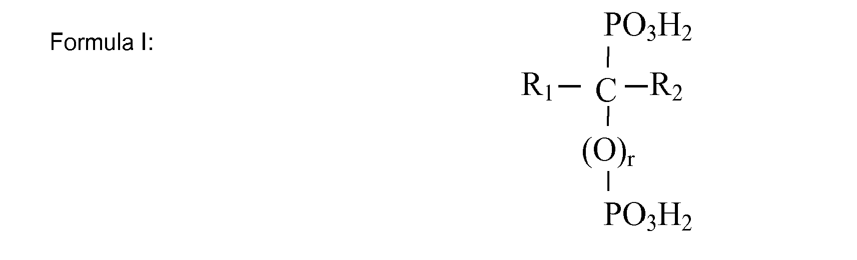 wherein
- R₁ is: a substituted or unsubstituted, saturated or partially unsaturated, linear, branched and/or cyclic, aliphatic or aromatic hydrocarbon with a chain of C₁ to C₂₀ carbon atoms, preferably C3 to C18, C4 to C15 or C6 to C13, and 0 to 5 heteroatoms, each heteroatom independently being N, O, P or S, wherein the heteroatoms can be located at any position within the hydrocarbon chain; or a primary, secondary or tertiary amino group, preferably of the structure N(R₄)₂ with each R₄ independently being H or a substituted or unsubstituted, saturated or partially unsaturated, linear, branched and/or cyclic, aliphatic or aromatic hydrocarbon with C₁ to C₁₀ carbon atoms and 0 to 2 heteroatoms, each heteroatom independently being N, O, P or S;
- R₂ is: H, OH, CH₃, C₂H₅ or N(R₃)₂, wherein each R₃ is independently from each other H, CH₃ or C₂H₅ ; and
- r is: 0 or 1.

A substituted hydrocarbon is a hydrocarbon wherein at least one hydrogen is replaced by a an atom different from hydrogen or by a group of atoms. In a preferred substituted hydrocarbon one or more hydrogen atoms are substituted by -OH; =O; a halogen, preferably F, Cl, Br or I; -CHO; -COOH; -OCₓH₂ₓ₊₁ with x being 1, 2, 3, or 4; -COOCₓH₂ₓ₊₁ with x being 1, 2, 3, or 4; an amine group or an amide group.

A partially unsaturated hydrocarbon is a hydrocarbon that comprises at least one C=C double bond or C≡C triple bond and at least one C-C single bond.

In a preferred embodiment of Formula I, R1 is:
R₁ = CₙH₂ₙR₅, wherein n is an integer from 4 to 15 and R₅ is H or NH₂; or

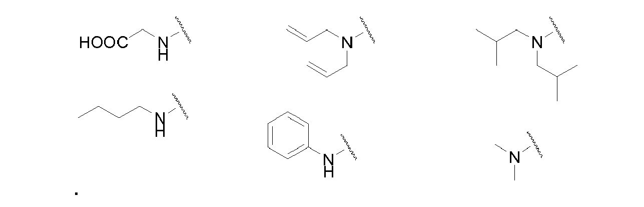

In a further preferred embodiment of Formula I, R1 is:
R1 = CₙH₂ₙR₅, wherein n is an integer from 5 to 13 and R₅ is H or NH₂ .

In compounds of Formula I of the present invention, R₂ can be H, OH, CH₃, NH₂, or N(CH₃)₂ .

Compounds of Formula II of the present invention comprise geminal bisphosphonates characterized by: 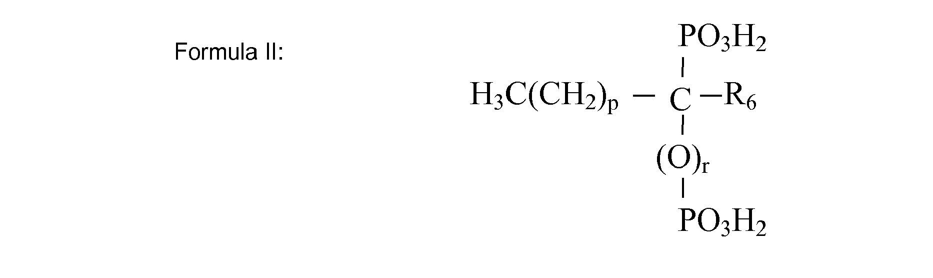 wherein
p is an integer from 4 to 12;
r is 0 or 1
R₆ = H, OH, NH₂ or N(CH₃)₂ .

Preferably R₆ is H, OH or NH₂, more preferably R₆ is OH or NH₂. The integer p can be from 4 to 12, preferably from 5 to 10.

Preferred compounds of Formula I or Formula II are the compounds:
H₃C(CH₂)₅C(PO₃H₂)₂H
H₃C(CH₂)₆C(PO₃H₂)₂H
H₃C(CH₂)₇C(PO₃H₂)₂H
H₃C(CH₂)₈C(PO₃H₂)₂H
H₃C(CH₂)₉C(PO₃H₂)₂H
H₃C(CH₂)₁₀C(PO₃H₂)₂H
H₃C(CH₂)₁₁C(PO₃H₂)₂H
H₃C(CH₂)₅C(PO₃H₂)₂OH
H₃C(CH₂)₆C(PO₃H₂)₂OH
H₃C(CH₂)₇C(PO₃H₂)₂OH
H₃C(CH₂)₈C(PO₃H₂)₂OH
H₃C(CH₂)₉C(PO₃H₂)₂OH
H₃C(CH₂)₁₀C(PO₃H₂)₂OH
H₃C(CH₂)₁₁C(PO₃H₂)₂OH
H₃C(CH₂)₅C(PO₃H₂)₂NH₂
H₃C(CH₂)₆C(PO₃H₂)₂NH₂
H₃C(CH₂)₇C(PO₃H₂)₂NH₂
H₃C(CH₂)₈C(PO₃H₂)₂NH₂
H₃C(CH₂)₉C(PO₃H₂)₂NH₂
H₃C(CH₂)₁₀C(PO₃H₂)₂NH₂
H₃C(CH₂)₁₁C(PO₃H₂)₂NH₂
H₃C(CH₂)₅C(PO₃H₂)₂N(CH₃)₂
H₃C(CH₂)₆C(PO₃H₂)₂N(CH₃)₂
H₃C(CH₂)₇C(PO₃H₂)₂N(CH₃)₂
H₃C(CH₂)₈C(PO₃H₂)₂N(CH₃)₂
H₃C(CH₂)₈C(PO₃H₂)₂N(CH₃)₂
H₃C(CH₂)₁₀C(PO₃H₂)₂N(CH₃)₂;
H₃C(CH₂)₁₁C(PO₃H₂)₂N(CH₃)₂;
H₃C(CH₂)₅C(PO₃H₂)(PO₄H₂)H
H₃C(CH₂)₆C(PO₃H₂)(PO₄H₂)H
H₃C(CH₂)₇C(PO₃H₂)(PO₄H₂)H
H₃C(CH₂)₈C(PO₃H₂)(PO₄H₂)H
H₃C(CH₂)₉C(PO₃H₂)(PO₄H₂)H
H₃C(CH₂)₁₀C(PO₃H₂)(PO₄H₂)H
H₃C(CH₂)₁₁C(PO₃H₂)(PO₄H₂)H
H₃C(CH₂)₅C(PO₃H₂)(PO₄H₂)OH
H₃C(CH₂)₆C(PO₃H₂)(PO₄H₂)OH
H₃C(CH₂)₇C(PO₃H₂)(PO₄H₂)OH
H₃C(CH₂)₈C(PO₃H₂)(PO₄H₂)OH
H₃C(CH₂)₉C(PO₃H₂)(PO₄H₂)OH
H₃C(CH₂)₁₀C(PO₃H₂)(PO₄H₂)OH
H₃C(CH₂)₁₁C(PO₃H₂)(PO₄H₂)OH
H₃C(CH₂)₅C(PO₃H₂)(PO₄H₂)NH₂
H₃C(CH₂)₆C(PO₃H₂)(PO₄H₂)NH₂
H₃C(CH₂)₇C(PO₃H₂)(PO₄H₂)NH₂
H₃C(CH₂)₈C(PO₃H₂)(PO₄H₂)NH₂
H₃C(CH₂)₉C(PO₃H₂)(PO₄H₂)NH₂
H₃C(CH₂)₁₀C(PO₃H₂)(PO₄H₂)NH₂
H₃C(CH₂)₁₁C(PO₃H₂)(PO₄H₂)NH₂
H₃C(CH₂)₅C(PO₃H₂)(PO₄H₂)N(CH₃)₂
H₃C(CH₂)₆C(PO₃H₂)(PO₄H₂)N(CH₃)₂
H₃C(CH₂)₇C(PO₃H₂)(PO₄H₂)N(CH₃)₂
H₃C(CH₂)₈C(PO₃H₂)(PO₄H₂)N(CH₃)₂
H₃C(CH₂)₉C(PO₃H₂)(PO₄H₂)N(CH₃)₂
H₃C(CH₂)₁₀C(PO₃H₂)(PO₄H₂)N(CH₃)₂; and/or
H₃C(CH₂)₁₁C(PO₃H₂)(PO₄H₂)N(CH₃)₂;
as well as all compounds described in Table 2.

Also preferred compounds are already known bisphosphonates of Formula I comprising the following compounds which have already been used for clinical applications, in particular for treatment, diagnosis and/or prophylaxis of osteoporosis:
- Etidronic acid (INN) or 1-hydroxyethane 1,1-diphosphonic acid (HEDP), systematic (IUPAC) name: (1-hydroxy-1-phosphono-ethyl)phosphonic acid;
- Tiludronic acid (INN; also known as tiludronate), systematic (IUPAC) name: {[(4-chlorophenyl)thio]methylene}bis(phosphonic acid). It is sold under the tradename Skelid®;
- Pamidronic acid (INN) or pamidronate disodium (USAN), pamidronate disodium pentahydrate is a nitrogen containing bisphosphonate, systematic (IUPAC) name: (3-amino-1-hydroxypropane-1,1-diyl)bis(phosphonic acid). It is marketed by Novartis under the brand name Aredia®;
- Neridronic acid (INN; the anion is called neridronate), systematic (IUPAC) name: (6-Amino-1-hydroxyhexane-1,1-diyl)bis(phosphonic acid);
- Olpadronic acid (INN; salt form *olpadronate*), systematic (IUPAC) name: [3-(dimethylamino)-1-hydroxypropane-1,1-diyl]bis(phosphonic acid);
- Alendronic acid (INN) or alendronate sodium (USAN, sold as Fosamax® by Merck), systematic (IUPAC) name: [4-amino-1-hydroxy-1-(hydroxy-oxido-phosphoryl)-butyl]phosphonic acid;
- Ibandronic acid (INN) or ibandronate sodium (USAN), marketed under the trade names Boniva, Bondronat and Bonviva, systematic (IUPAC) name: {1-hydroxy-3-[methyl(pentyl)amino]propane-1,1-diyl}bis(phosphonic acid)
- Risedronic acid (INN) or risedronate sodium (USAN, trade name Actonel®), systematic (IUPAC) name: (1-hydroxy-1-phosphono-2-pyridin-3-yl-ethyl)phosphonic acid;
- Zoledronic acid (INN) or zoledronate (marketed by Novartis under the trade names Zometa®, Zomera®, Aclasta® and Reclast®), systematic (IUPAC) name: (1-hydroxy-2-imidazol-1-yl-1-phosphono-ethyl)phosphonic acid; and
- 2,3-Dicarboxypropan-1,1-Diphosphonat (DPD).

Since compounds of Formula I and Formula II inhibit enzyme activity of aSMase in vitro and in vivo, these compounds can be used as inhibitors of aSMase. In particular a compound of Formula I or II can be used for inhibition of acid sphingomyelinase enzyme activity in vitro. The term "in vitro" refers to any use or method not practised on the human or animal body. Such a use encompasses the use of compounds of Formula I or II in a cellular or cell-free assay for aSMase activity.

Compounds of Formula I or II may be used as a medicament, in particular as a medicament for treatment, diagnosis and/or prophylaxis of a disease associated with altered, elevated or unwanted aSMase enzyme activity.

It has already been documented that aSMase enzyme activity plays a crucial role in a number of diseases. Diseases which have already been associated with aSMase activity comprise e.g.:
- infectious diseases like bacterial infections, e.g. with infection with Neisseria gonnorhoeae (H. Grassme, E. Gulbins, B. Brenner, K. Ferlinz, K. Sandhoff, K. Harzer, F. Lang, T. F. Meyer, Cell 1997, 91, 605);
- lung diseases like acute lung injury (ALI) or acute respiratory distress syndrome (ARDS), lung oedema, (R. Goggel, S. Winoto-Morbach, G. Vielhaber, Y. Imai, K. Lindner, L. Brade, H. Brade, S. Ehlers, A. S. Slutsky, S. Schutze, E. Gulbins, S. Uhlig, Nat Med 2004, 10, 155);
- pulmonary emphysema (I. Petrache, V. Natarajan, L. Zhen, T. R. Medler, A. T. Richter, C. Cho, W. C. Hubbard, E. V. Berdyshev, R. M. Tuder, Nat Med 2005, 11, 491);
- infections, e.g. bacterial infections, during cystic fibrosis (V. Teichgraber, M. Ulrich, N. Endlich, J. Riethmuller, B. Wilker, C. C. De Oliveira-Munding, A. M. van Heeckeren, M. L. Barr, G. von Kurthy, K. W. Schmid, M. Weller, B. Tummler, F. Lang, H. Grassme, G. Doring, E. Gulbins, Nat Med 2008, 14, 382);
- Morbus Wilson (P. A. Lang, M. Schenck, J. P. Nicolay, J. U. Becker, D. S. Kempe, A. Lupescu, S. Koka, K. Eisele, B. A. Klarl, H. Rubben, K. W. Schmid, K. Mann, S. Hildenbrand, H. Hefter, S. M. Huber, T. Wieder, A. Erhardt, D. Haussinger, E. Gulbins, F. Lang, Nat Med 2007, 13, 164);
- atherosclerosis, coronary heart disease, cardiovascular diseases (C. M. Devlin, A. R. Leventhal, G. Kuriakose, E. H. Schuchman, K. J. Williams, I. Tabas, Arterioscler Thromb Vasc Biol 2008, 28, 1723);
- diabetes type II (Gorska, M., Baranczuk, E., and Dobrzyn, A. (2003) Horm. Metab. Res. 35,506-507; Straczkowski, M., Kowalska, I., Baranowski, M., Nikolajuk, A., Otziomek, E., Zabielski, P., Adamska, A., Blachnio, A., Gorski,J., and Gorska, M. (2007) Increased skeletal muscle ceramide level in men at risk of developing type 2 diabetes. Diabetologia 50, 2366-2373);
- major depression (Kornhuber, J., Medlin, A., Bleich, S., Jendrossek, V., Henkel, A. W., Wiltfang, J., and Gulbins, E. (2005) High activity of acid sphingomyelinase in major depression. J. Neural. Transm. 112,1583-1590);
- Alzheimer disease (Han, X. (2005) Lipid alterations in the earliest clinically recognizable stage of Alzheimer's disease: implication of the role of lipids in the pathogenesis of Alzheimer's disease. Curr. Alzheimer Res. 2, 65-77);
- Niemann-Pick disease (E. Schuchman, R. J. Desnick, in The Metabolic Basis of Inherited Disease (Eds.: C. Scriver, W. Sly, D. Valle), McGraw Hill, New York, 2001, pp. 3589; J. Q. Fan, Trends Pharmacol Sci 2003, 24, 355.; E. Schuchman, R. J. Desnick, **2005**, p. WO 2005/051331.)
- Cancer (T.Kirkegaard et al. Nature manuscript in revision)

Niemann-Pick Disease (NPD) Type A and B is one of several known lysosomal storage diseases. It is a rare, recessively inherited disease caused by mutations in the gene coding for the acid sphingomyelinase (aSMase) leading to a partial loss of functional enzyme in the lysosomes. As a consequence, sphingomyelin, a major constituent of eukaryotic plasma membranes and the substrate of acid sphingomyelinase can not be degraded, but accumulates within the lysosomes of the affected organs of NPD patients. Depending on residual acid sphingomyelinase (aSMase) activity, individuals with an inborn defect in the aSMase gene, develop Type A or B NPD. The infantile Type A (aSMase activity less than 3% of normal) is the more severe form and is characterized by neuronal involvement and death by the age of 2 or 3 years. Type B NPD (less than 6% of normal aSMase activity) is the juvenile non-neuronopathic form of the disease and patients may survive into adulthood. Enzymatic activity above a threshold level of about 10% usually results in a complete or at least sufficient sphingomyelin turnover without any pathological phenotype. Thus, only a small increase in residual enzyme activity could have a significant impact on disease development and on life quality of NPD patients. Especially the milder forms of lysosomal storage disorders like NPD Type B are likely to be protein misfolding diseases, because alterations within the active site of an enzyme normally results in a complete loss of activity. A new, but very promising approach to treat lysosomal storage disorders is the use of small molecule substrate analogues or competitive inhibitors as chemical chaperones. The benefit of chemical chaperones is to protect variant enzymes from being degraded by the proteasome and to facilitate their transport to the lysosomes, thereby rescuing enzymatic activity. The rationale of this approach is the fact that variant lysosomal enzymes produced as a consequence of an inborn genetic mutation in the aSMase gene might be active in the acid environment of the lysosomes if only they could get there. Chemical chaperone mediated protection of variant enzymes occurs probably due to stabilisation of the native state fold of an otherwise misfolded enzyme by binding to its active site. Because of the very different chemical environments in the ER and in the lysosomes, some variant enzymes, which do not fold properly in the ER might retain partial or even full catalytic activity within the acidic chemical environment of the lysosomes. After an enzyme-inhibitor complex has reached the lysosome, the inhibitor is replaced by the accumulated substrate competing with the inhibitor to bind to the active site of the enzyme. Thus, paradoxically, an inhibitor of an enzyme *in vitro* can act as an enzyme activator *in vivo.*

The concept of chemical chaperones for the treatment of lysosomal storage disorders has first been described by Fan et al. for Fabry disease. Application of 1-deoxy-galactonojirimycin (DGJ) an inhibitor of α-galactosidase A (α-Gal A) effectively enhanced activity of this enzyme in Fabry lymphoblasts and in transgenic mice overexpressing a human Fabry variant of α-Gal A. Furthermore, injection of galactose, a much weaker inhibitor of α-Gal A to a cardiac Fabry patient resulted in considerable regression of pathology. It revealed that DGJ is able to stimulate α-Gal A seven- to eight-fold in cells when used at sub-inhibitory intracellular concentrations.

In fact, it was demonstrated that a potent inhibitor provides an effective chaperone, whereas less potent inhibitors require higher concentrations to achieve the same effect. This notion is most important, since potent inhibitors are expected to have therapeutic effects at lower concentrations that interact more specifically with the enzyme. By contrast, higher concentrations of moderately potent inhibitors are more likely to cross-react with other proteins.

Besides DGJ, which is in pre-clinical development, the concept has proven so far in cell culture with other substances for two further lysosomal storage disorders. Up to now, the exact mechanism by which chemical chaperones exhibit their function still remains to be elucidated. Recently, it has been found out that variant glucocerebrosidase characterized by destabilization of domains other than the catalytic domain is not amenable to stabilization by active site directed chemical chaperones. It is very likely that this observation reflects a general principle for chemical chaperones. Probably, stabilization of non active site domains may only be achieved by specifically designed molecules.

The potential for chemical chaperones to treat Niemann-Pick Disease has recently been outlined in WO 2005/051331.

Heat shock protein 70 (Hsp70) promotes the survival of cells, e.g. cancer cells, by stabilizing lysosomes, a hallmark of stress-induced cell death. (J. Nylandsted, M. Gyrd-Hansen, A. Danielewicz et al., J Exp Med 200 (4), 425 (2004)). In cancer, a portion of Hsp70 translocates to the lysosomal compartment. It could be shown that Hsp70 stabilizes lysosomes by enhancing the activity of lysosomal acid sphingomyelinase. The pharmacological and genetic inhibition of aSMase effectively reverts the Hsp70-mediated stabilization of lysosomes (T. Kirkegaard et al. Nature, in revision). Thus, inhibitors of aSMase sensitize cancer cells and tumours to chemo- or radiotherapy and therefore can be used in treatment, diagnosis and/or prophylaxis of cancer.

Thus, compounds of Formula I or II can be used in treatment, diagnosis and/or prophylaxis of infectious diseases, bacterial infections, infection with Neisseria gonnorhoeae, infections associated with cystic fibrosis, bacterial infections associated with cystic fibrosis, lung diseases, acute lung injury, acute respiratory distress syndrome, lung oedema, pulmonary emphysema, cystic fibrosis, Morbus Wilson, atherosclerosis, coronary heart disease, cardiovascular diseases, diabetes type II, depression, Alzheimer disease and/or Niemann-Pick disease and cancer.

Preferably a compound of Formula II is used as a medicament.

A compound of Formula I or II can be used for the preparation of a medicament for inhibition of acid sphingomyelinase enzyme activity.

A compound of Formula I or II can be used for the preparation of a medicament for treatment, diagnosis and/or prophylaxis of infectious diseases, bacterial infections, infection with Neisseria gonnorhoeae, infections associated with cystic fibrosis, bacterial infections associated with cystic fibrosis, lung diseases, acute lung injury, acute respiratory distress syndrome, lung oedema, pulmonary emphysema, cystic fibrosis, Morbus Wilson, atherosclerosis, coronary heart disease, cardiovascular diseases, diabetes type II, depression, Alzheimer disease and/or Niemann-Pick disease and cancer.

The present invention also refers to a method of treatment, diagnosis or prophylaxis of a disease associated with aSMase activity, comprising the administration of an effective amount of a compound of Formula I or Formula II. An effective amount is an amount that yields to a measurable result with regard to treatment, diagnosis or prophylaxis of a disease associated with aSMase activity.

### FIGURES

- Figure 1: shows that the aSMase inhibitor **7c** (0.1µM) inhibits dexamethasone (Dex)- induced apoptosis in HepG2 cells. The data refer to absorbance in a DNA- fragmentation ELISA.

- Figure 2: shows that the aSMase inhibitor **7c** reduces PAF-induced pulmonary edema in isolated, ventilated and perfused rat lungs (IPL). Weight gain was measured 10 min after PAF-donation (5 nM).
- Figure 3: shows that the aSMase inhibitor 7c does not significantly inhibit PP1 activity when used in concentrations up to 2 µM.

### EXAMPLES

### Example 1: Bisphosphonates and mixed phosphonate/phosphate compounds of Formula I and Formula II are potent and selective inhibitors of aSMase

A collection of (bis)phosphonates that contained some compounds structurally-related to Ptdlns3,5P₂ were tested for their ability to inhibit aSMase. When theses substances were initially tested at 20 µM concentrations, it was found surprisingly that these compounds inhibited aSMase very potently (Tables 1 & 2). Among these substances, the geminal α-aminobisphosphonate **7b** turned out to be about one order of magnitude more potent than Ptdlns3,5P₂. Furthermore, **7b** in comparison with **7a,** only consists of two additional methylene units, which leads to a dramatic increase in inhibitory potency.

**Table 1. Inhibition of aSMase by the intial phosphonate collection**

| | | | | |
|---|---|---|---|---|
| | 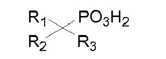 | | | |

| Com poun d | R₁ | R₂ | R₃ | Inhibition [%] at 20 µM ^{[a]} |
|---|---|---|---|---|
| **1** | 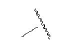 | H | H | 16 |
| **2** | 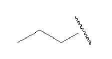 | H | H | 2 |
| **3** | 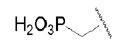 | NH₂ | H | 47 |
| **4** | 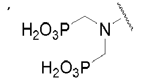 | H | H | 0 |
| **5** | 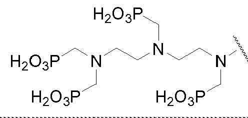 | H | H | -5 |

**Table 2. Inhibition of aSMase by the intial bisphosphonate collection**

| | | | |
|---|---|---|---|
| | 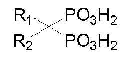 | | |

| Compound | R₁ | R₂ | Inhibition [%] at 20 µM |
|---|---|---|---|
| **6** | 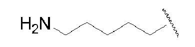 | OH | 54 |
| **7a** | 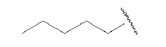 | NH₂ | 92 |
| **7b** | 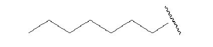 | NH₂ | 93 |
| **8** | 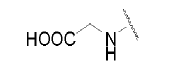 | CH₃ | 62 |
| **9** | 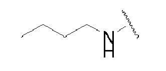 | H | 76 |
| **10** | 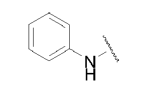 | H | 8 |
| **11** | 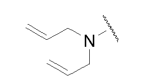 | H | 32 |
| **12** | 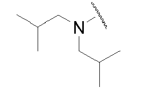 | H | 2 |
| **13** | 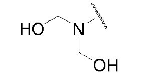 | H | 36 |
| **14** | 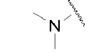 | H | 24 |

In order to gain a deeper insight into the structure activity relationship, a battery of 15 additional bisphosphonates were synthesized harbouring different functional groups at the α-carbon and displaying lipid-tails of different length, respectively (Scheme 1). Most of the syntheses were one- or two-step procedures yielding up to gram-amounts of the inhibitors according to well-established protocols ( a) D. A. Nicholson, H. Vaughn, J. Org. Chem. 1971, 36, 3843. b) L. M. Nguyen, E. Niesor, C. L. Bentzen, J Med Chem 1987, 30, 1426. c) G. R. Kieczykowski, R. B. Jobson, D. G. Melillo, D. F. Reinhold, V. J. Grenda, I. Shinkai, J. Org Chem. 1995, 60, 8310. d) D. V. Griffiths, J. M. Hughes, J. W. Brown, J. C. Caesar, S. P. Swetnam, S. A. Cumming, J. D. Kelly, Tetrahedron 1997, 53, 17815. e) S. H. Szajnman, E. L. Ravaschino, R. Docampo, J. B. Rodriguez, Bioorg Med Chem Lett 2005, 15, 4685.). On the basis of this new collection of compounds, it could be shown that inhibition correlates with the length of the lipid tail (this correlation is true as long as the substances are well-soluble) and that a functional group with free electron pairs at the α-carbon (-NH₂ more strongly than -OH) leads to an additionally increased inhibition of acid sphingomyelinase, when compared to the H-bisphosphonates **15a-d.** Moreover, zoledronic acid **20,** a widely-used drug against osteoporosis showed a marked inhibition of aSMase with an IC₅₀ value of approximately 5 µM (Table 3).

**Table 3. Inhibition of aSMase by the synthesized bisphosphonates^{[a]}**

| Entry | IC₅₀[µM] | Entry | IC₅₀[µM] | Entry | IC₅₀[µM] |
|---|---|---|---|---|---|
| **7a** | 4.66 ± 1.07 | **15c** | 0.30 ± 0.05 | **18c** | 0.07 ± 0.01 |
| **7b** | 0.04±0.01 **15d** | | 0.17 ± 0.04 | **18d** | 6.80 ± 2.40 |
| **7c** | 0.02 ± 0.00 | **16** | > 100 | **18e** | 1.95 ± 0.22 |
| **7d** | 0.29 ± 0.09 | **17** | 0.35 ± 0.06 | **19a** | 9.50 ± 4.00 |
| **15a** | 0.35 ± 0.08 **18a** | | 0.16 ± 0.04 | **19b** | 0.18 ± 0.03 |
| **15b** | 0.31 ± 0.12 **18b** | | 0.08 ± 0.01 | **20** | 5.08 ± 0.74 |

| | | | | | |
|---|---|---|---|---|---|
| [a] The IC₅₀ value for inhibition of nSMase was > 100 µM for all compounds. | | | | | |

Bisphosphonates are known to form bidentate complexes with Me²⁺-ions like Ca²⁺, Zn²⁺ and Mg²⁺. With an additional hydroxyl or amine group, even more stable tridentate complexes can be formed. In fact, α-amino substitution leads to more stable complexes than an α-hydroxyl substitution, suggesting that aSMase inhibition also correlates with the tendency of the compounds to form complexes with the Zn²⁺ residing in the reactive center of the aSMase. It is noteworthy that aSMase, both in its lysosomal and its secreted form, is a Zn²⁺-dependent enzyme. However, the lysosomal variant is not inhibited by EDTA and not stimulated by Zn²⁺, which can be explained by abundance of Zn²⁺ in the lysosomes, whereas the secreted variant is stimulated by Zn²⁺. In order to characterize the aSMase- inhibiting bisphosphonates with regard to their metal-binding properties, compound 7c was tested in presence of millimolar concentrations of Ca²⁺, Mg²⁺ or Zn²⁺, respectively. The inhibitory activity was not significantly diminished by the metal ions. ***Scheme 1**.* Synthesis of bisphosphonates. Reagents and conditions: a) NaH, Toluene, 60 °C, 16 h b) HCI, reflux, 16 h c) P(OMe)₃, 0 °C, 2 h d) HP(OMe)₂, *n*Bu₂NH, 0 °C, 16 h e) H₃PO₃, MsOH, then PCl₃, 90 °C, 16 h f) PCl₃/H₃PO₃, 70°C, 12 h, then H₂O, 2h

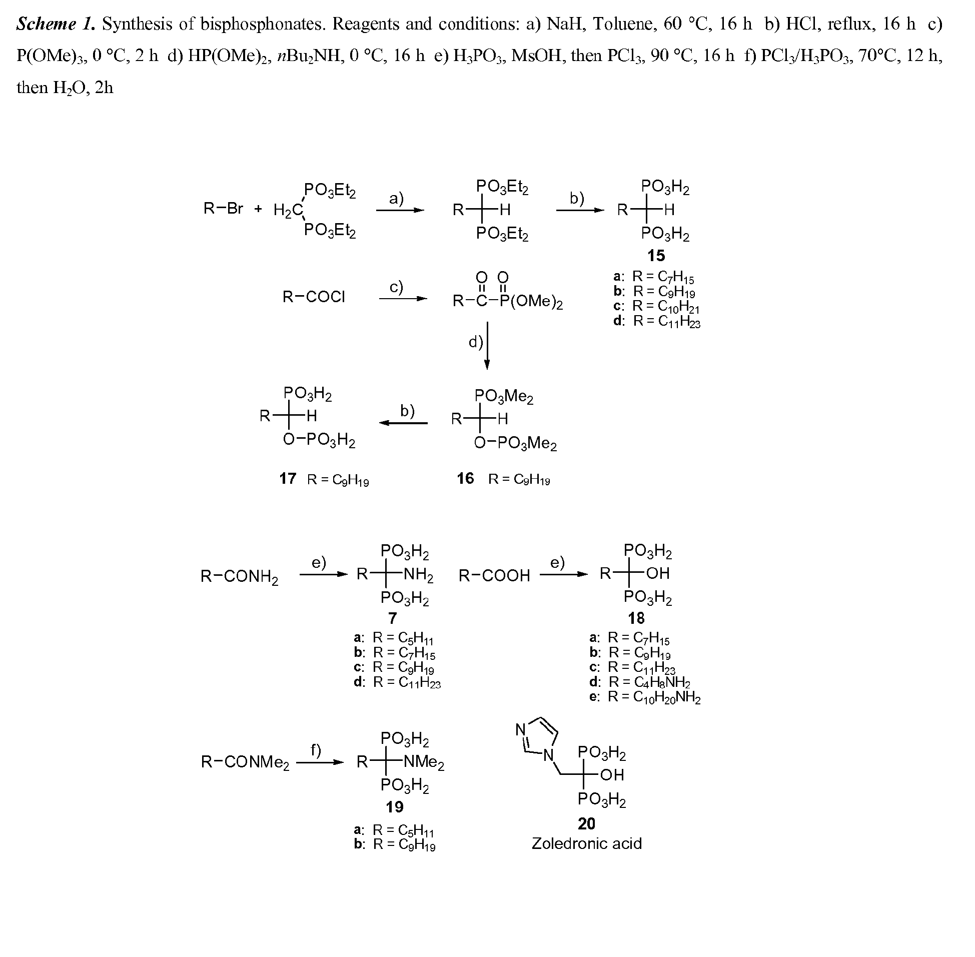

In addition, virtually all substances were tested for an inhibition of the Mg²⁺-dependent nSMase, without observing any substantial inhibition of this isoenzyme at concentrations up to 100 µM, clearly indicating that inhibition of aSMase is not only very potent, but is at the same time highly selective against nSMase.

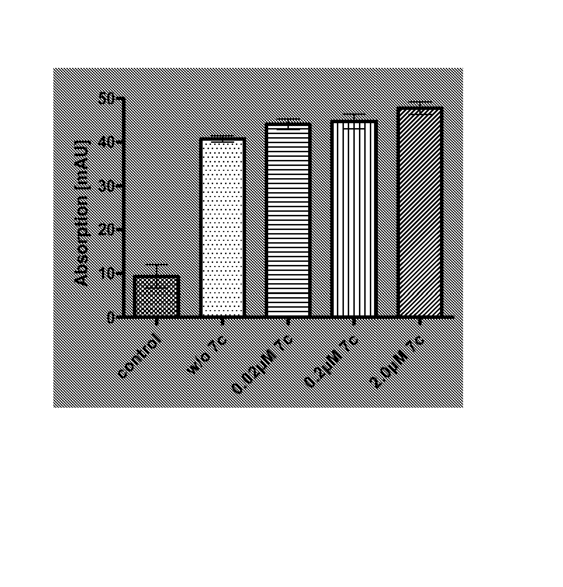

Moreover, the aSMase inhibitor **7c** was tested for any inhibitory effect on the Ser/Thr phosphatase 1 (PP1), which - like the phosphodiesterase domain of aSMase - belongs to a family of dimetal-containing phosphoesterases. The PP1 enzyme was not inhibited by **7c,** even at a concentration of 2 µM, which shows that this aSMase inhibitor is selective vs. PP1 (see Figure 3).

To test for the influence of the negatively charged residues, the mixed phosphate/phosphonate compounds **16** and **17** were synthesized and tested. Whereas the mixed phosphate/phosphonate compound **17** is as active as its bisphosphonate analogue **15b,** the methyl ester **16** is totally inactive towards aSMase, suggesting that aSMase inhibition is dependent on the metal complexing properties of the bisphosphonates.

### Example 2: Inhibition of aSMase activity can efficiently inhibit apoptosis

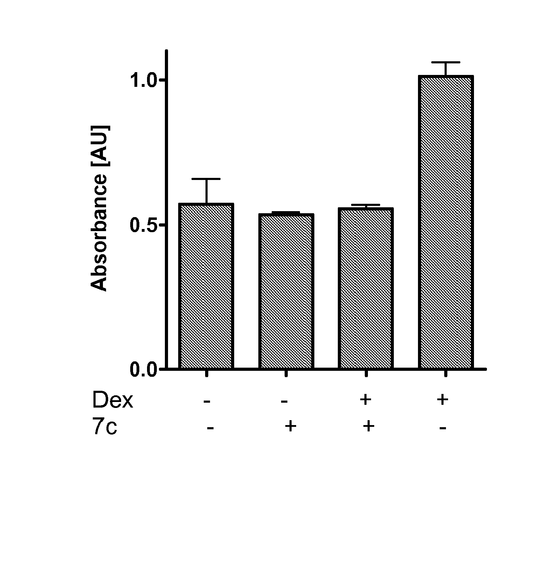

HepG2 liver cells were treated with dexamethasone (10⁻⁸ M) in order to induce apoptosis, 0.1 µM of the aSMase inhibitor **7c** efficiently inhibited apoptosis, as measured with a commercially-available DNA-fragmentation ELISA (Figure 1).

### Example 3: Inhibition of aSMase activity can inhibit pulmonary oedema

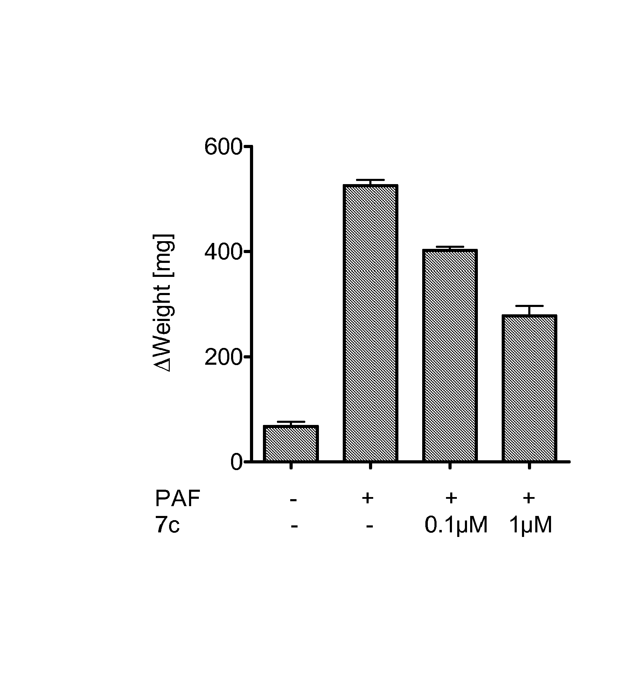

Encouraged by the high biological activity in cultured cells and because of the evident pharmacological interest in potent aSMase inhibitors for the treatment of lung diseases, it was examined whether inhibition of aSMase is also able to reduce PAF-induced pulmonary oedema, similar to the unspecific and indirect aSMase inhibitor imipramine (S. Uhlig, E. Gulbins, Am. J. Respir. Crit. Care Med. 2008, 178, 1100). Indeed, addition of **7c** to the perfusate was concentration-dependently reduced oedema formation in isolated, ventilated and perfused rat lungs (IPL, shown in Figure 2). Like imipramine (10 µM), the inhibitor **7c** attenuated but not completely prevented oedema formation in this model.

The simple bisphosphonate **7c** is the most potent aSMase inhibitor found so far. It is more than 5.000fold selective against the Mg²⁺-dependent isoenzyme nSMase and selective against the dimetal-containing remote aSMase-homologue Ser/Thr protein phosphatase 1. The compound, which easily can be synthesized in gram-scale is also active in cell culture and efficiently protects HepG2 cells from dexamethasone-induced apoptosis.

### Experimental Procedures

**Enzyme assays:** Crude preparations containing aSMase or nSMase were made from stripped rat brains, as described before. The micellar nSMase assays using ¹⁴C-labeled sphingomyelin as a substrate were performed as described before (V. Wascholowski, A. Giannis, Angew. Chem., 2006, 118, 841; Angew Chem Int Ed 2006, 45, 827). The fluorescent aSMase assay was performed in a 384-well-plate using the HMU-PC (6-Hexadecanoylamino-4-methylumbelliferyl- phosphorylcholine) substrate. Reaction mixtures consisted of 13.3µL HMU-PC, 13.3µL reaction-buffer (100mM NaOAc, pH 5.2, 0.2% (w/v) Na-TC, 0.02% (w/v), 0.2% (v/v) Triton X-100) and 13.3µL enzyme preparation. Inhibitors were added in various concentrations and the reactions were incubated for 3 hours at 37°C in a plate reader (FLUOstar OPTIMA, BMG labtech). The fluorescence of HMU (6-Hexadecanoylamino-4-methylumbelliferone) was measured (excitation 380nm, emission 460nm) in real time. Assays using the radio-labelled sphingomyelin gave the same results.

**Compound libraries and syntheses:** all described compounds were verified using ¹H-, ¹³C- and ³¹P-NMR and MS, respectively. The syntheses were accomplished as described before (a) D. A. Nicholson, H. Vaughn, J. Org. Chem. 1971, 36, 3843. b) L. M. Nguyen, E. Niesor, C. L. Bentzen, J Med Chem 1987, 30, 1426. c) G. R. Kieczykowski, R. B. Jobson, D. G. Melillo, D. F. Reinhold, V. J. Grenda, I. Shinkai, J. Org Chem. 1995, 60, 8310. d) D. V. Griffiths, J. M. Hughes, J. W. Brown, J. C. Caesar, S. P. Swetnam, S. A. Cumming, J. D. Kelly, Tetrahedron 1997, 53, 17815. e) S. H. Szajnman, E. L. Ravaschino, R. Docampo, J. B. Rodriguez, Bioorg Med Chem Lett 2005, 15, 4685).

**Apoptosis assay:** First, the kinetics of DNA fragmentation after dexamethasone-donation was measured in the lysate and in the supernatant, respectively. Between 6h and 8h, there was a steep increase in absorbance in the probes from the supernatant, which is typical for apoptosis (data not shown). The apoptosis assay was performed according to the manufacturer's protocol (Roche cat. No. 11585045). Briefly, cells were harvested and suspended in culture medium (2×10⁵ cells/ml) containing BrdU labelling solution (10 µM final concentration) and plated in a 96-well cell culture dish at ~1x10⁴ cells per well. After 16h, cells were washed and new media was added. Then, cells were treated with 10⁻⁸ M of dexamethasone and 0.1 µM of **7c,** respectively. After 7 hours of incubation, 100 µl of the supernatant was collected and added to a 96well plate containing immobilized anti-BrdU antibody. After incubation, removal of the supernatant and extensive washing, the secondary antibody and the TMB substrate were added and absorbance was measured at 370 nm (FLUOstar OPTIMA, BMG labtech). The experiment was performed in quintuplicate.

**PAF-induced pulmonary edema:** Female Wistar rats (weight 220 to 250g) were kept on a standard laboratory chow and water *ad libitum.* Rat lungs were prepared, perfused and ventilated essentially as described (S. Uhlig, E. Gulbins, Am. J. Respir. Crit. Care Med. 2008, 178, 1100). Briefly, lungs were perfused through the pulmonary artery at a constant hydrostatic pressure (12 cm H₂O) with Krebs-Henseleit-buffer containing 2% albumin, 0.1% glucose and 0.3% HEPES. Edema formation was assessed by continuously measuring the weight gain of the lung. In this model, platelet-activating factor causes rapid edema formation that is in part dependent on acid sphingomyelinase. **7c** was dissolved in buffer and added to the buffer reservoir 10 min prior to PAF (5 nMol) administration. Isolated perfused rat lungs were perfused for 30 min before **7c** was added to the perfusate. 10 min later 5 nMol PAF was added as a bolus and weight gain was followed for 10 min. Data are shown as mean ± SD from 4 independent experiments in each group. Statistics: 0.1µM **7c:** p<0.01 vs PAF alone; 1µM **7c:** p<0.01 vs. PAF alone and vs. 0.1 µM **7c**/PAF (Tukey's Test).

**PP1 assay:** The protein phosphatase 1 (PP1, *New England Biolabs* P0754L) activity was assayed in a reaction mixture of 50µL according to the manufacturer's conditions, but containing only 1 % (500 µM) of the recommended amount of *p*-nitrophenylphosphate (PNPP, *New England Biolabs* P0757L). In a preceding experiment the K_{M} for PNPP was determined to be 3 mM (data not shown), which is in agreement with the manufacturer's statement (Km = 0.5 to 10 mM for all phosphatases). Briefly, the substrate and various inhibitor concentrations were added to the reaction buffer containing 1mM MnCl₂, 50mM HEPES, 100mM NaCl, 0.1mM EGTA, 2mM dithiothreitol, 0.025% Tween 20 at pH 7.5. The reaction was initiated by addition of PP1 (1.25U). After 6 min the reaction was quenched by addition of 10 µl of 0.5M EDTA-solution (pH 8). The amount of the formed product, p-nitrophenol, was determined by measuring the absorbance at 405nm (Nanodrop). The control was composed as described above, including 0.2 µM **7c** but with heat-denatured enzyme. All measurements were done at least in triplicate.

### Procedures for the Synthesis of Previously Unknown Substances (16, 17 and 19 a/b:)

### Procedure for preparation of 1-Dimethylaminodecyl-1,1-bisphosphonicacid (19b)

*N*,*N*-Dimethyldecanamide (1.0g, 5.02mmol) was slowly added to an initially stirred mixture of phosphorus trichloride (1.0ml, 11.4mmol) and phosphorous acid (0.42g, 5.12mmol). The mixture was heated at 70°C for 2h. After cooling the excess phosphorous trichloride was decanted off and the residue hydrolyzed by the careful addition of plenty of water. This mixture was left to stir for at least 2h, filtered, and the filtrate evaporated to dryness under reduced pressure. The precipitate was taken up in 20ml of water and heated at 100°C for 1 h, followed by filtration of the hot solution. The water was evaporated and the desired product was isolated as a colorless solid (1.73g, quant).**¹H N**MR **(300MHz, D₂O):** δ = 0.84 (t, *J*= 6.72Hz, 3H), 1.26 (m, 8H), 1.31 (d, *J* = 3.94Hz, 4H), 1.55 (dd, *J* = 2.28, 4.34Hz, 2H), 2.00 (m, 2H), 3.063 (s, 6H)ppm. **¹³C NMR (75MHz, D₂O):** δ = 13.46, 22.16, 28.69, 28.75, 28.92, 29.14, 30.06, 31.34, 31.41, 41.80, 70.04 (t, *J* = 108.65Hz, 1C)ppm. **³¹P NMR (121 MHz, D₂O):** δ = 3.71 ppm. HRMS: m/z calcd. for C₁₂H₂₈NO₆P₂: 344.1397, found: 344.1389.

### Procedure for preparation of 1-Dimethylaminodecyl-1,1-bisphosphonicacid (19a)

*N*,*N*-Dimethylhexanamide (1.5g, 10.6mmol) was slowly added to an initially stirred mixture of phosphorus trichloride (2.8ml, 32.2mmol) and phosphorous acid (1.15g, 14.0mmol). The mixture was heated at 70°C for 2h. After cooling the excess phosphorous trichloride was decanted off and the residue hydrolyzed by the careful addition of plenty of water. This mixture was left to stir for at least 2h, filtered, and the filtrate evaporated to dryness under reduced pressure. The precipitate was taken up in 20ml of water and heated at 100°C for 1 h, followed by filtration of the hot solution. The water was evaporated and the desired product was isolated as a colorless solid (1.64g, 54%). **¹H NMR (300MHz, D₂O):** δ = 0.83 (t, *J* = 6.26Hz, 3H), 1.27 (tt, *J* = 3.50, 7.24Hz, 4H), 1.50 (qd, *J* = 7.04, 6.98, 8.70Hz, 2H), 1.98 - 2.04 (m, 2H), 3.03 (s, 6H)ppm. **¹³C NMR (75MHz, D₂O):** δ = 13.22, 21.58, 23.00, 28.93, 31.54, 42.07, 69,18ppm. **³¹P NMR (121MHz, D₂O):** δ = 4.66ppm. HRMS: m/z calcd. for C₈H₂₂NO₆P₂: 290.0917, found: 290.0904.

### Procedure for preparation of Decyl-1-dimethylphosphate-1-dimethylphospohonate (16)

Decanoylchloride (4g, 21.0mmol) was placed in a mechanically stirred reaction flask and cooled to 0°C. Trimethylphosphite (2.60g, 21.0mmol) was added drop wise with rapid stirring (gas evolution). After addition was complete the reaction mixture was allowed to warm up at room temperature. The reaction mixture was evaporated under reduced pressure. To the colourless oil was added dimethylphosphite (1.15g, 10.5mmol) and ether (50ml), followed by an addition of di-n-butylamine (0.14g, 1.05mmol) and cooling to 0°C. The reaction mixture was allowed to warm up at room temperature and was stirring over night. Purification of the crude product was purified by silica gel chromatography (dichloromethane/methanol 20:1) and gave the desired product in 16% (1.33g) yield. **¹H NMR (300MHz, CDCl₃):** δ = 0.67 - 0.76 (m, 3H), 1.11 (s, 12H), 1.25 - 1.45 (m, 2H), 1.65 - 1.80 (m, 2H), 1.82 - 1.90 (m, 1H), 3.55 - 3.73 (m, 12H)ppm. **¹³C NMR (75MHz, CDCl₃):** δ = 13.91, 22.48, 24.99, 25.14, 28.94, 29.10, 29.15, 29.29, 30.65, 31.70, 53.21, 53.29, 54.33, 54.41, 71.65, 71.74, 73.89, 73.99ppm. **³¹P NMR (121MHz, CDCl₃):** δ = 1.57. 1.73. 22.95, 23.11ppm. HRMS: m/z calcd. for C₁₄H₃₃O₇P₂: 375.1696, found: 375.1685.

### Procedure for preparation of Decyl-1,1-phosphate phosphonate (17)

The Dimethylphophonate dimethylphosphateesters of compound 16 (0.35g, 0.94mmol) was hydrolyzed by refluxing for 8h with an excess of concentrated hydrochloride acid. The acid was evaporated and the desired product was isolated as colorless oil in 99% (0.29g, 0.91 mmol) yield. **¹H NMR (300MHz, D₂O):** δ = 0.57 (t, *J* = 6.37Hz, 3H), 0.90-1.10 (m, 12H), 1.22 - 1.43 (m, 2H), 1.48 - 1.64 (m, 2H), 4.14 (s, 1H)ppm. **¹³C NMR (75MHz, D₂O):** δ = 13.56, 22.40, 25.14, 25.28, 29.24, 29.33, 29.63, 30.46, 31.81, 72.31, 72.39, 74.48, 74.57ppm. **³¹P NMR (121MHz, D₂O):** δ = 0.22, 20.43ppm. HRMS: m/z calcd. for C₁₀H₂₅O₇P₂: 319.1070, found: 319.1070.

## Claims

1. Compound of Formula I for use in treatment, diagnosis and/or prophylaxis of infectious diseases, bacterial infections, infection with Neisseria gonorrhoea, infections associated with cystic fibrosis, bacterial infections associated with cystic fibrosis, lung diseases, acute lung injury, acute respiratory distress syndrome, lung oedema, pulmonary emphysema, cystic fibrosis, Morbus Wilson, atherosclerosis, coronary heart disease, cardiovascular diseases, diabetes type II, depression, Alzheimer disease, cancer and/or Niemann-Pick disease,
with 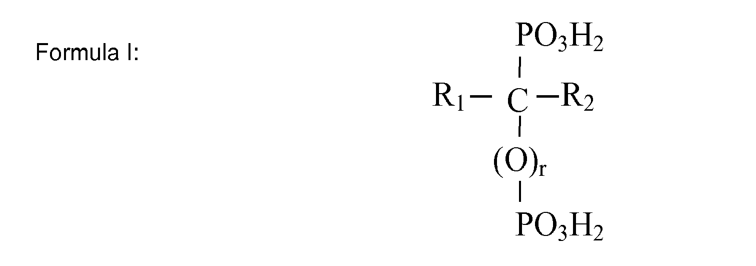 wherein
R₁ = a substituted or unsubstituted, saturated or partially unsaturated, linear, branched and/or cyclic, aliphatic or aromatic hydrocarbon with a chain of C₁ to C₂₀ carbon atoms and 0 to 5 heteroatoms, each heteroatom independently being N, O, P or S;
or
a primary, secondary or tertiary amino group, preferably of the structure N(R₄)₂ with each R₄ independently being H or a substituted or unsubstituted, saturated or partially unsaturated, linear, branched and/or cyclic, aliphatic or aromatic hydrocarbon with C₁ to C₁₀ carbon atoms and 0 to 2 heteroatoms each heteroatom independently being N, O, P or S;
R₂ = H, OH, CH₃, C₂H₅ or N(R₃)₂, wherein each R₃ is independently from each other H, CH₃ or C₂H₅ ;
r = 0 or 1.

2. Compound of claim 1, wherein
R₁ = CₙH₂ₙR₅, wherein n is an integer from 4 to 15 and R₅ is H or NH₂; or

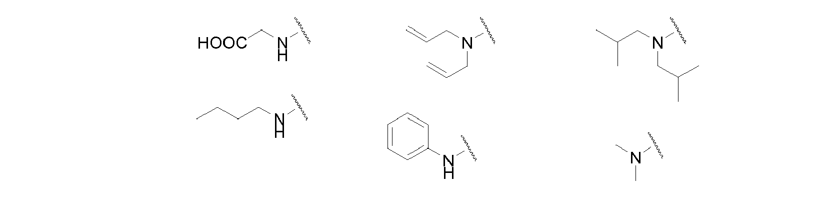

3. Compound of one of claims 1 to 2, wherein
R1 = CₙH₂ₙR₅, wherein n is an integer from 5 to 13 and R₅ is H or NH₂ .

4. Compound of one of claims 1 to 3, wherein
R₂ = H, OH, CH₃, NH₂, or N(CH₃)₂ .

5. Compound of Formula II, with 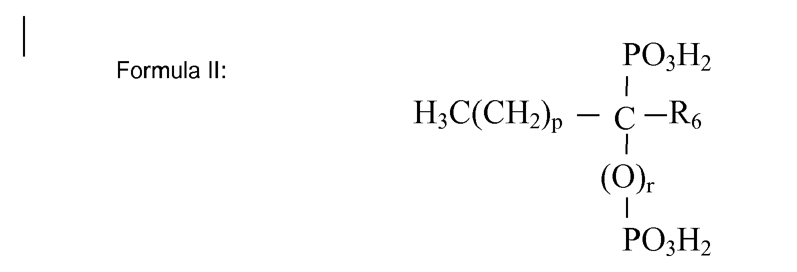 ; wherein
p is an integer from 4 to 12;
r is 0 or 1
R₆ = H, OH, NH₂ or N(CH₃)₂ .

6. Compound of claim 5, wherein the compound is:
H₃C(CH₂)₅C(PO₃H₂)₂H
H₃C(CH₂)₆C(PO₃H₂)₂H
H₃C(CH₂)₇C(PO₃H₂)₂H
H₃C(CH₂)₈C(PO₃H₂)₂H
H₃C(CH₂)₉C(PO₃H₂)₂H
H₃C(CH₂)₁₀C(PO₃H₂)₂H
H₃C(CH₂)₁₁C(PO₃H₂)₂H H₃C(CH₂)₅C(PO₃H₂)₂OH H₃C(CH₂)₆C(PO₃H₂)₂OH H₃C(CH₂)₇C(PO₃H₂)₂OH H₃C(CH₂)₈C(PO₃H₂)₂OH H₃C(CH₂)₉C(PO₃H₂)₂OH H₃C(CH₂)₁₀C(PO₃H₂)₂OH H₃C(CH₂)₁₁C(PO₃H₂)₂OH H₃C(CH₂)₅C(PO₃H₂)₂NH₂ H₃C(CH₂)₆C(PO₃H₂)₂NH₂ H₃C(CH₂)₇C(PO₃H₂)₂NH₂ H₃C(CH₂)₈C(PO₃H₂)₂NH₂ H₃C(CH₂)₉C(PO₃H₂)₂NH₂ H₃C(CH₂)₁₀C(PO₃H₂)₂NH₂ H₃C(CH₂)₁₁C(PO₃H₂)₂NH₂ H₃C(CH₂)₅C(PO₃H₂)₂N(CH₃)₂ H₃C(CH₂)₆C(PO₃H₂)₂N(CH₃)₂ H₃C(CH₂)₇C(PO₃H₂)₂N(CH₃)₂ H₃C(CH₂)₈C(PO₃H₂)₂N(CH₃)₂ H₃C(CH₂)₈C(PO₃H₂)₂N(CH₃)₂ H₃C(CH₂)₁₀C(PO₃H₂)₂N(CH₃)₂; H₃C(CH₂)₁₁C(PO₃H₂)₂N(CH₃)₂ H₃C(CH₂)₅C(PO₃H₂)(PO₄H₂)H H₃C(CH₂)₆C(PO₃H₂)(PO₄H₂)H H₃C(CH₂)₇C(PO₃H₂)(PO₄H₂)H H₃C(CH₂)₈C(PO₃H₂)(PO₄H₂)H H₃C(CH₂)₉C(PO₃H₂)(PO₄H₂)H H₃C(CH₂)₁₀C(PO₃H₂)(PO₄H₂)H H₃C(CH₂)₁₁c(PO₃H₂)(PO₄H₂)H H₃C(CH₂)₅C(PO₃H₂)(PO₄H₂)OH H₃C(CH₂)₆C(PO₃H₂)(PO₄H₂)OH H₃C(CH₂)₇C(PO₃H₂)(PO₄H₂)OH H₃C(CH₂)₈C(PO₃H₂)(PO₄H₂)OH H₃C(CH₂)₉C(PO₃H₂)(PO₄H₂)OH H₃C(CH₂)₁₀C(PO₃H₂)(PO₄H₂)OH H₃C(CH₂)₁₁C(PO₃H₂)(PO₄H₂)OH H₃C(CH₂)₅C(PO₃H₂)(PO₄H₂)NH₂ H₃C(CH₂)₆C(PO₃H₂)(PO₄H₂)NH₂ H₃C(CH₂)₇C(PO₃H₂)(PO₄H₂)NH₂
H₃C(CH₂)₈C(PO₃H₂)(PO₄H₂)NH₂
H₃C(CH₂)₉C(PO₃H₂)(PO₄H₂)NH₂
H₃C(CH₂)₁₀C(PO₃H₂)(PO₄H₂)NH₂
H₃C(CH₂)₁₁C(PO₃H₂)(PO₄H₂)NH₂
H₃C(CH₂)₅C(PO₃H₂)(PO₄H₂)N(CH₃)₂
H₃C(CH₂)₆C(PO₃H₂)(PO₄H₂)N(CH₃)₂
H₃C(CH₂)₇C(PO₃H₂)(PO₄H₂)N(CH₃)₂
H₃C(CH₂)₈C(PO₃H₂)(PO₄H₂)N(CH₃)₂
H₃C(CH₂)₉C(PO₃H₂)(PO₄H₂)N(CH₃)₂
H₃C(CH₂)₁₀C(PO₃H₂)(PO₄H₂)N(CH₃)₂; and/or
H₃C(CH₂)₁₁C(PO₃H₂)(PO₄H₂)N(CH₃)₂.

7. Compound of claims 5 and 6 for use as medicament.

8. Compound of claims 5 and 6 for use in treatment, diagnosis and/or prophylaxis of infectious diseases, bacterial infections, infection with Neisseria gonnorhoeae, infections associated with cystic fibrosis, bacterial infections associated with cystic fibrosis, lung diseases, acute lung injury, acute respiratory distress syndrome, lung oedema, pulmonary emphysema, cystic fibrosis, Morbus Wilson, atherosclerosis, coronary heart disease, cardiovascular diseases, diabetes type II, depression, Alzheimer disease, cancer and/or Niemann-Pick disease.

9. Use of a compound of Formula I or II as inhibitor of acid sphingomyelinase, with 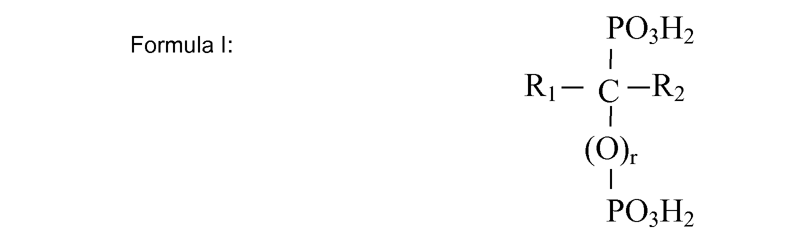 wherein
R₁ = a substituted or unsubstituted, saturated or partially unsaturated, linear, branched and/or cyclic, aliphatic or aromatic hydrocarbon with a chain of C₁ to C₂₀ carbon atoms and 0 to 5 heteroatoms, each heteroatom independently being N, O, P or S;
or
a primary, secondary or tertiary amino group, preferably of the structure N(R₄)₂ with each R₄ independently being H or a substituted or unsubstituted, saturated or partially unsaturated, linear, branched and/or cyclic, aliphatic or aromatic hydrocarbon with C₁ to C₁₀ carbon atoms and 0 to 2 heteroatoms each heteroatom independently being N, O, P or S;
R₂ = H, OH, CH₃, C₂H₅ or N(R₃)₂, wherein each R₃ is independently from each other H, CH₃ or C₂H₅ ;
r = 0 or 1; and 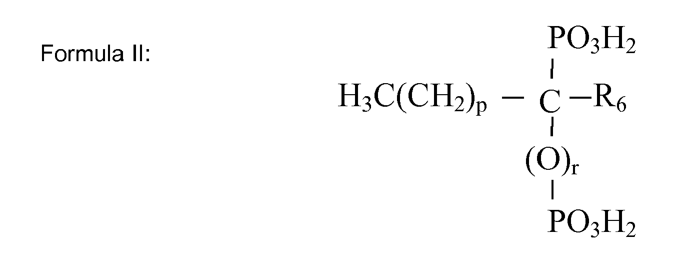 wherein
p is an integer from 4 to 12;
r is 0 or 1
R₆ = H, OH, NH₂ or N(CH₃)₂ .

10. Use of claim 9, wherein acid sphingomyelinase is inhibited in vitro.

11. Use of a Compound of Formula I or II for the preparation of a medicament for inhibition of acid sphingomyelinase,
with 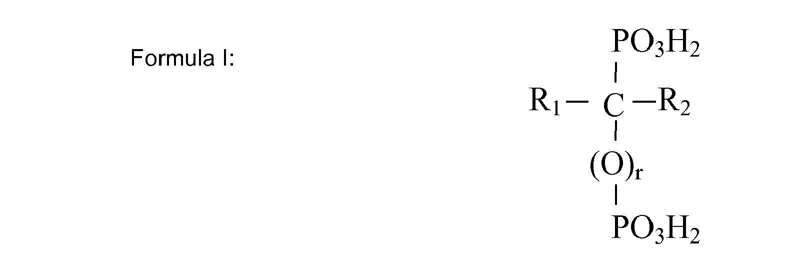 wherein
R₁ = a substituted or unsubstituted, saturated or partially unsaturated, linear, branched and/or cyclic, aliphatic or aromatic hydrocarbon with a chain of C₁ to C₂₀ carbon atoms and 0 to 5 heteroatoms, each heteroatom independently being N, O, P or S;
or
a primary, secondary or tertiary amino group, preferably of the structure N(R₄)₂ with each R₄ independently being H or a substituted or unsubstituted, saturated or partially unsaturated, linear, branched and/or cyclic, aliphatic or aromatic hydrocarbon with C₁ to C₁₀ carbon atoms and 0 to 2 heteroatoms each heteroatom independently being N, O, P or S;
R₂ = H, OH, CH₃, C₂H₅ or N(R₃)₂, wherein each R₃ is independently from each other H, CH₃ or C₂H₅ ;
r = 0 or 1; and 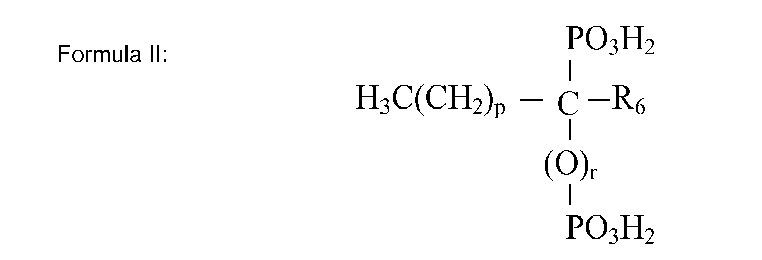
wherein
p is an integer from 4 to 12;
r is 0 or 1
R₆ = H, OH, NH₂ or N(CH₃)₂ .

12. Use of claim 11, wherein the medicament is for treatment, diagnosis and/or prophylaxis of infectious diseases, bacterial infections, infection with Neisseria gonnorhoeae, infections associated with cystic fibrosis, bacterial infections associated with cystic fibrosis, lung diseases, acute lung injury, acute respiratory distress syndrome, lung oedema, pulmonary emphysema, cystic fibrosis, Morbus Wilson, atherosclerosis, coronary heart disease, cardiovascular diseases, diabetes type 11, depression, Alzheimer disease, cancer and/or Niemann-Pick disease.
